# FASCICULE DE BREVET EUROPEEN

(11) **EP 0 626 173 B1**
(45) Date de publication et mention de la délivrance du brevet: **04.12.1996**
(21) Numéro de dépôt: 94401131.1
(22) Date de dépôt: 24.05.1994
(51) Int. Cl.: A61K 31/71, A61K 9/00

(54) **Formulation d'aérosol à base de fusafungine**
Aerosolformulierung enthaltend Fusafungin
Aerosol composition containing fusafungine

(30) Priorité: 25.05.1993 FR 9306179
(43) Date de publication de la demande: 30.11.1994
(73) Titulaire: ADIR ET COMPAGNIE, F-92415 Courbevoie Cédex (FR)
(72) Inventeur: Cuine, Alain, F-45800 St Jean de Braye (FR); Morisseau, Serge, F-45770 Saran (FR)

(56) Documents cités:
- FR-M- 1 084
- US-A- 3 305 445
- DATABASE WPI Week 6800, Derwent Publications Ltd., London, GB; AN 66-15198F (00) & CA-A-700 411 (BIOFARMA SA)
- DATABASE WPI Week 6800, Derwent Publications Ltd., London, GB; AN 66-15198F (00)

## Description

La présente invention se rapporte à un nouvel aérosol pharmaceutique à base de fusafungine, son procédé de préparation et son utilisation à des fins thérapeutiques.

La fusafungine est un antibiotique local actif sur de nombreuses souches parmi lesquelles : streptocoques du groupe A, pneumocoques, staphylocoques, certaines souches de neisseria, certains germes anaérobies, candida et mycoplasma pneumoniae.

La fusafungine présente également des propriétés antiinflammatoires.

Ce type de propriétés rend la fusafungine particulièrement intéressante à utiliser dans le traitement local des inflammations et infections de la muqueuse de l'oropharynx et des voies aériennes au cours des sinusites, rhinites, rhinopharyngites, amygdalites, suites d'amygdalectomie, laryngites, trachéites, bronchites...

De par les indications dans laquelle elle est utile au malade, la fusafungine est essentiellement administrée par voie orale ou nasale.

Plus particulièrement depuis une trentaine d'années, la fusafungine est présentée - au corps médical et au public - sous forme d'un aérosol dont la formule renferme, outre le principe actif, un mélange d'excipients qui sont : la saccharine, l'essence composée, l'essence de Néroli, l'éthanol, le benzododécinium et le myristate d'isopropyle. Cette composition contient en outre un propulseur qui est du dichlorodifluorométhane.

Cette forme pharmaceutique, actuellement sur le marché, présente la caractéristique particulièrement avantageuse d'être une solution de principe actif dans un mélange d'excipients à pouvoir solvant constitué par éthanol, essence de Néroli, essence composée, myristate d'isopropyle, et bromure de benzododécinium. Cette caractéristique, assez peu fréquente pour un aérosol, permet aux particules d'aérosol administrées d'atteindre toutes les voies respiratoires, y compris les alvéoles pulmonaires, assurant un maximum d'efficacité thérapeutique.

Un autre avantage, résultant également du fait que cet aérosol est une solution, est que la fabrication d'une telle formulation est facilitée : les phénomènes de démixion, floculation, sédimentation, manque d'homogénéité rencontrés avec les suspensions n'existent plus.

Toutefois, pour l'industriel qui a la charge de fabriquer puis de commercialiser cette spécialité, une telle formulation présente l'inconvénient de renfermer, outre le principe actif, sept excipients distincts nécessaires à l'obtention d'une composition présentant les caractères requis pour la conservation et l'administration chez l'homme d'une spécialité pharmaceutique.

On conçoit aisément la lourdeur qui s'attache pour l'industriel à utiliser sept excipients pour une formulation renfermant un seul principe actif. Il suffit d'une rupture d'approvisionnement de la part de l'un des fournisseurs en excipients et l'industriel ne peut plus fabriquer la composition pharmaceutique. Or la diffusion de cette spécialité de par ses indications obéit à un processus en grande partie saisonnier. On conçoit aisément que la rupture d'approvisionnement, même pendant une courte période, d'un seul de ces excipients risque de provoquer d'importantes conséquences :
- pour l'industriel, perte de la vente de l'équivalent d'une année de production d'une de ses plus importantes spécialités (en nombre d'unités vendues),
- pour de nombreux malades, impossibilité de trouver un médicament très efficace et bien toléré.

C'est dans cet esprit que des recherches ont été menées afin de diminuer le nombre des composants de cette formulation à base de fusafungine, tout en lui conservant les qualités inhérentes à une composition pharmaceutique sous forme d'aérosol.

De façon surprenante, il est apparu que le remplacement du propulseur 12 par le propulseur 134a encore appelé 1,1,1,2-tétrafluoroéthane permet de réduire à un seul le nombre des excipients indispensables à une formulation à base de fusafungine présentée sous forme d'aérosol.

Dès lors la composition pharmaceutique se réduit à trois constituants :
- le principe actif, en l'ocurrence la fusafungine,
- le propulseur, indispensable à tout aérosol, en l'ocurrence le propulseur 134a,
- un seul autre excipient choisi parmi diméthylisosorbide ou préférentiellement, myristate d'isopropyle.

Tout d'abord l'un des avantages principaux de l'ancienne formulation, actuellement sur le marché, est conservé : on obtient toujours une solution malgré la suppression de plusieurs excipients à pouvoir solvant, et ceci est surprenant eu égard aux caractères physicochimiques de la fusafungine. L'efficacité thérapeutique maximale et la facilité de fabrication de la spécialité pharmaceutique sont conservées.

Un autre intérêt de la présente invention est que la composition pharmaceutique obtenue par utilisation du propulseur 134a peut être modulable en fonction des approvisionnements en matières premières. En particulier, si on remplace dans la composition pharmaceutique actuellement commercialisée et qui renferme les sept excipients, le propulseur 12 par le propulseur 134a, on obtient une composition nouvelle qui présente également les caractères de stabilité requis pour une composition pharmaceutique, au même titre que la composition ne renfermant pour seuls excipients que le propulseur 134a et le myristate d'isopropyle ou le diméthylisosorbide. Des essais concluants ont également été réalisés par suppression d'un seul ou de plusieurs des autres excipients suivants : l'éthanol, le bromure de benzododécinium, l'eucalyptol, l'essence de Néroli.

De façon générale, la nouvelle forme pharmaceutique peut être présentée dans des bidons dont la taille varie entre 5 et 200 ml. Le principe actif, la fusafungine, entre dans une proportion de 0,01 à 5% en poids de la formulation selon l'invention. Le propulseur entre dans la composition des formulations selon l'invention dans une proportion comprise entre 55 et 95% en poids, préférentiellement entre 70 et 80% en poids de la formulation. La fusafungine est incorporée à un excipient choisi parmi diméthylisosorbide ou préférentiellement myristate d'isopropyle, excipient qui entre dans la composition dans une proportion comprise entre 10 et 40% en poids, préférentiellement entre 20 et 30% en poids.

Les excipients que l'on trouve dans l'ancienne formulation peuvent toujours être ajoutés :

| | |
|---|---|
| Saccharine | 0 à 1% en poids |
| Ethanol | 0 à 5% en poids |
| Bromure de benzododécinium | 0 à 0,1% en poids |
| Essence de Néroli | 0 à 2% en poids |
| Eucalyptol | 0 à 2% en poids |
| Compositions aromatiques | 0 à 5% en poids. |

Les exemples suivants indiquent des cas particuliers de l'invention et ne la limitent en aucune façon.

### EXEMPLE 1 :

Fusafungine : 50 mg
Myristate d'isopropyle Q.S.P. : 5 ml
Propulseur 134a : 15 ml.

### EXEMPLE 2 :

Fusafungine : 50 mg
Diméthylisosorbide : Q.S.P. 5 ml
Propulseur 134a : 15 ml.

Les formulations précédentes sont préparées par mélange des différents constituants et agitation. La quantité requise du mélange est introduite dans un flacon adéquat et une valve distributrice doseuse pour aérosol y est introduite. Le propulseur 134a est incorporé sous pression.

## Revendications

1. Composition pharmaceutique sous forme d'aérosol comprenant comme seuls ingrédients un principe actif : la fusafungine, un excipient choisi parmi myristate d'isopropyle ou diméthylisosorbide et un propulseur : le 1,1,1,2-tétrafluoroéthane ou propulseur 134a.

2. Composition pharmaceutique selon la revendication 1 dans laquelle l'excipient est le myristate d'isopropyle.

3. Composition pharmaceutique selon la revendication 1 dans laquelle la fusafungine entre dans une proportion comprise entre 0,01 et 5 % du poids total de la formulation.

4. Composition pharmaceutique selon la revendication 1 dans laquelle le 1,1,1,2-tétrafluoroéthane entre dans une proportion comprise entre 55 et 95 % du poids total de la formulation.

5. Composition pharmaceutique selon la revendication 2 dans laquelle le myristate d'isopropyle entre dans une proportion comprise entre 10 et 40 % du poids total de la formulation.

6. Composition pharmaceutique selon la revendication 2, qui est :
Fusafungine : 50 mg
Myristate d'isopropyle Q.S.P. : 5 ml
Propulseur 134a : 15ml.

7. Composition pharmaceutique selon la revendication 1 dans laquelle l'excipient est le diméthylisosorbide.

8. Composition pharmaceutique selon la revendication 7 qui est :
Fusafungine : 50 mg
Diméthylisosorbide Q.S.P. : 5 ml
Propulseur 134a : 15 ml.

9. Spécialité pharmaceutique visant à l'administration de fusafungine à un patient par voie orale ou nasale comprenant un récipient d'aérosol équipé d'une valve distributrice doseuse, le récipient d'aérosol contenant une composition pharmaceutique selon l'une quelconque des revendications 1 à 7.

10. Procédé de préparation d'une spécialité pharmaceutique qui consiste à remplir un récipient d'aérosol avec une composition pharmaceutique selon l'une des revendications 1 à 7 et à équiper le récipient d'aérosol d'une valve distributrice doseuse.

## Claims

1. Pharmaceutical composition in aerosol form comprising as sole ingredients an active ingredient: fusafungine, an excipient selected from isopropyl myristate and dimethyl isosorbide, and a propellant: 1,1,1,2-tetrafluoroethane, or propellant 134a.

2. Pharmaceutical composition according to claim 1 in which the excipient is isopropyl myristate.

3. Pharmaceutical composition according to claim 1 in which fusafungine is present in an amount of between 0.01 and 5 % of the total weight of the formulation.

4. Pharmaceutical composition according to claim 1 in which 1,1,1,2-tetrafluoroethane is present in an amount of between 55 and 95 % of the total weight of the formulation.

5. Pharmaceutical composition according to claim 2 in which isopropyl myristate is present in an amount of between 10 and 40 % of the total weight of the formulation.

6. Pharmaceutical composition according to claim 2 which is :
fusafungine : 50 mg
isopropyl myristate quantity sufficient for : 5 ml
propellant 134a : 15 ml.

7. Pharmaceutical composition according to claim 1 in which the excipient is dimethyl isosorbide.

8. Pharmaceutical composition according to claim 7 which is :
fusafungine : 50 mg
dimethyl isosorbide quantity sufficient for : 5 ml
propellant 134a : 15 ml.

9. Pharmaceutical specialty intended for the administration of fusafungine to a patient by the oral or nasal route, comprising an aerosol container fitted with a dose dispensing valve, the aerosol container containing a pharmaceutical composition according to any one of claims 1 to 7.

10. A process for the preparation of a pharmaceutical specialty which comprises filling an aerosol container with a pharmaceutical composition according to any one of claims 1 to 7 and fitting the aerosol container with a dose dispensing valve.

## Patentansprüche

1. Pharmazeutische Aerosolzubereitung enthaltend als einzige Bestandteile einen Wirkstoff: Fusafungin, ein Trägermaterial ausgewählt aus Isopropylmyristat oder Dimethylisosorbid und ein Treibmittel: 1,1,1,2-Tetrafluorethan oder Treibmittel 134a.

2. Pharmazeutische Zubereitung nach Anspruch 1, worin das Trägermaterial Isopropylmyristat ist.

3. Pharmazeutische Zubereitung nach Anspruch 1, worin das Fusafungin in einem Anteil zwischen 0,01 und 5 % des Gesamtgewichts der Zubereitung vorhanden ist.

4. Pharmazeutische Zubereitung nach Anspruch 1, worin 1,1,1,2-Tetrafluorethan in einem Anteil zwischen 55 und 95% des Gesamtgewichts der Zubereitung vorhanden ist.

5. Pharmazeutische Zubereitung nach Anspruch 2, worin das Isopropylmyristat in einem Anteil zwischen 10 und 40 % des Gesamtgewichts der Zubereitung vorhanden ist.

6. Pharmazeutische Zubereitung nach Anspruch 2, nämlich:
Fusafungin: 50 mg
Isopropylmyristat: ad 5 ml
Treibmittel 134a: 15 ml.

7. Pharmazeutische Zubereitung nach Anspruch 1, worin das Trägermaterial Dimethylisosorbid ist.

8. Pharmazeutische Zubereitung nach Anspruch 1, nämlich:
Fuasfungin: 50 mg
Dimethylisosorbid: ad 5 ml
Treibmittel 134a: 15 ml.

9. Pharmazeutische Spezialität zur Verabreichung von Fusafungin auf oralem oder nasalem Wege an einen Patienten, umfassend einen Aerosolbehälter, der mit einem Dosierventil versehen ist, welcher Aerosolbehälter eine pharmazeutische Zubereitung nach einem der Ansprüche 1 bis 7 enthält.

10. Verfahren zur Herstellung einer pharmazeutischen Spezialität, welches darin besteht, einen Aerosolbehälter mit einer pharmazeutischen Zubereitung nach einem der Ansprüche 1 bis 7 zu befüllen und den Aerosolbehälter mit einem Dosierventil zu versehen.
